# EUROPEAN PATENT APPLICATION

(11) **EP 3 556 845 A1**
(43) Date of publication of application: **23.10.2019**
(21) Application number: 18168523.1
(22) Date of filing: 20.04.2018
(51) Int. Cl.: C12M 3/06, C12M 1/34

(54) **A METHOD AND DEVICE FOR TRANSFECTING CELLS**

(71) Applicant: Cellix Limited, 12 Dublin (IE)
(72) Inventor: Kashanin, Dmitry, Dublin, D12 (IE); Shvets, Igor, Dublin, D12 (IE); Williams, Vivienne, Dublin, D12 (IE)
(74) Representative: Purdylucey Intellectual Property

(57) **Abstract**

A method of transfecting a population of cells, comprising the steps of treating the population of cells to disrupt a cell membrane of at least some of the cells, and introducing foreign material into at least some of the cells through the disrupted cell membranes. The cell membranes are disrupted by passing a cell-containing carrier liquid along a microfluidic channel having a cross-sectional dimension greater a cross-sectional dimension of the cells and comprising a flow obstacle configured to abruptly change a flow vector of the cells and/or carrier liquid in the microfluidic channel from a first direction to a second direction. The abrupt change in flow vector of the cells is configured to cause transfection-competent disruption of the cell membrane of at least some of the cells due to impact of the cells with the flow obstacle and/or with other cells.

## Description

### Field of the Invention

The present invention relates to a method and device for transfecting cells. Also contemplated are methods and devices for preparing a population of cells for transfection by physically disrupting the cell membranes of the cells.

### Background to the Invention

In the past decade genetic engineering of living cells and organisms has become a hugely important field of research with exciting applications envisaged in medicine, farming, production of animals, production of food and other areas. The momentum in this field has accelerated with the advent of the CRISPR/Cas9 genome-editing platform [D. Carroll, Genome engineering with targetable nucleases, Annu. Rev. Biochem. 83 (2014) 409-439; M. Jinek, K. Chylinski, I. Fonfara, M. Hauer, J.A. Doudna, E. Charpentier, A programmable dual-RNA-guided DNA endonuclease in adaptive bacterial immunity, Science 337 (2012) 816-821; M. Jinek, A. East, A. Cheng, S. Lin, E. Ma, J. Doudna, RNA-programmed genome editing in human cells, Elife 2 (2013) e00471; L. Cong, F.A. Ran, D. Cox, S. Lin, R. Barretto, N. Habib, P.D. Hsu, X. Wu, W. Jiang, L.A. Marraffini, F. Zhang, Multiplex genome engineering using CRISPR/Cas systems, Science (80-.) 339 (2013); S. Kim, D. Kim, S.W. Cho, J. Kim, J.-S. Kim, Highly efficient RNA-guided genome editing in human cells via delivery of purified Cas9 ribonucleoproteins, Genome Res. 24 (2014) 1012-1019; and S. Lin, B. Staahl, R.K. Alia, J.A. Doudna, Enhanced homology-directed human genome engineering by controlled timing of CRISPR/Cas9 delivery, Elife 3 (2014) e04766]. This system requires a complex of Cas9 endonuclease protein with a gene-targeting guide RNA (gRNA) to introduce double-strand DNA breaks at specific locations in the genome. The breaks are then repaired by the error-prone non-homologous end joining pathway, resulting in insertions and/or deletions which disrupt the targeted locus. CRISPR/Cas9 is the most vibrant tool in genetic engineering at present. However, there are also other methods, both emerging and current. In all of these methods it is essential to deliver biological material (e.g. DNA, RNA, gRNA, ribonucleoprotein (RNP), protein, virus, etc) from the outside of the cell across the cell membrane to the interior of the cell, the process called transfection. It is important that the cell stays alive after the completion of the transfection to initiate the process induced by the transfected material within the cell. Therefore, developing methods of transfection has gained much importance in recent years.

Further information on the importance of transfer of genome-editing and therapeutic proteins across the cell membrane is provided in the following publication and references therein [J. A. Zuris, D.B. Thompson, et. al., Efficient Delivery of Genome-Editing Proteins In Vitro and In Vivo Nat Biotechnol. 2015 January; 33(1): 73-80. doi:10.1038/nbt.3081]. For review of the methods of transfer of CAS9 and guide RNA (gRNA) across the cell membrane one could refer to [M.A. DeWitt, J.E. Corn, D. Carroll, Genome editing via delivery of Cas9 ribonucleoprotein. Methods 121-122 (2017) 9-15].

Generally, the cell membrane protects interior of the cell from the introduction of alien biological material (i.e. transfecting the cell) and therefore the transfection has to disrupt temporarily this barrier function of the membrane. There are several ways of transfecting a cell including using chemical transfection methods [P. Midoux, C. Pichon, J-J. Yaouanc, PA. Jaffres, Chemical vectors for gene delivery: a current review on polymers, peptides and lipids containing histidine or imidazole as nucleic acids carriers. Br. J. Pharmacol. 2009; 157:166-178. PubMed: 19459843; J.J. Cronican, et al. Potent delivery of functional proteins into Mammalian cells in vitro and in vivo using a supercharged protein. ACS Chem. Biol. 2010; 5:747-752 PubMed: 20545362; D.B. Thompson, R. Villaseñor, B.M. Dorr, M. Zerial, D.R. Liu, Cellular uptake mechanisms and endosomal trafficking of supercharged proteins. Chem. Biol. 2012; 19:831-843 PubMed: 22840771], viral vectors [M.A. Kay, J.C. Glorioso, L. Naldini, Viral vectors for gene therapy: the art of turning infectious agents into vehicles of therapeutics. Nat. Med. 2001; 7:33-40. PubMed: 11135613], electroporation [A.M. Bodles-Brakhop, R. Heller, R. Draghia-Akli, Electroporation for the Delivery of DNA-based Vaccines and Immunotherapeutics: Current Clinical Developments. Mol. Ther. 2009; 17:585-592. PubMed: 19223870], cell squeezing and mechanical processing of cells in a mix with beads.

Electroporation appears to be the most commonly used method for transfection. Although it is not directly relevant to the present invention, we briefly mention representative inventions and publications on transfection by electroporation. US patent 5,186,800 to Dower describes electroporation for transfer of nucleic acids into cells. US patent 7,078,227 to Bebee et. al. describes cuvette for electroporation of frozen cell as they thaw. US patent 9,382,510 to Chen describes electroporation of a monolayer of cells in a cuvette. Publication of a patent application US2006/0224192 (Dimmer) describes device of in vivo electroporation for delivery of chemical agents e.g. into a tumour. US patent 8,222,014 (Firth et al.) describes an apparatus and method for planar electroporation. US patent 6,846,306 (Hass et al.) describes apparatus and method for electroporation of single cells. US patent 7,521,224 (Johnson) describes an electroporation array assembly comprising the electrodes, electronics for generating voltage pulses and means for supplying the fluid to be transfected into the cells. US patent 9,816,086 (Lee et al) describes an apparatus and method for intracellular delivery of biological liquid into individual cells in microfluidic format. US patent 8,828,736 (Perroud et al.) describes a microelectroporation plate combined with optically transparent wells for genomic screening. According to the inventors, this device allows for higher throughput and lower costs of screening. US patent 7,456,012 (Ryttsen et al) describes method and device for conducting electrophoresis is well plates. US patent 6,878,538 (Walters et al) described an apparatus and method for electroporation in conventional well plates. US patents 8,206,903 and 8,921,041, both to Wang et al., describe apparatus and method for conducting electroporation of cells on a membrane. The cells are recruited on a membrane from a suspension and the said membrane has electrodes integrated on it.

The two methods of transfection: cell squeezing and mechanical processing of cells in a mix with beads, are most relevant to this invention and are described in more detail. The advantage of mechanical methods is in their universality and simplicity. The methods do not rely on foreign biological and chemical materials and therefore can be applied to a range of cells and biological materials. The processing of cells with assistance of beads normally requires loading cells with beads (glass, silica) and the material to be transfected into a closed vessel and subjecting it to a mechanical action (spinning, shaking, vibratring, etc). The advantage is in the simplicity of the method and large volume of cells that can be processed at the same time. The disadvantage is in the lack of the level of control of the process and selectivity. After the completion of the spinning and shaking of the vessel, it will contain a mixture of the dead cells, lysed cells with their content spilled into the vessel, cells with membrane open for transfection, cells with damaged membrane that have low viability. Separating from these the cells that have been transfected and have acceptable viability is not straightforward. Besides all the cells may need to be separated from the beads after completion of the transfection. The method of cell squeezing relies on transfer of cells under high hydraulic pressure via an opening that has size smaller than the size of the cell. During the transfer, the cells deform, become elongated and their membrane becomes permeable for the transfection. This is achieved as a result of a large pressure differential applied across the opening. This method is described in the patent applications [US2018/0016539 (Ding et al); US2016/0193605 (Sharei et al); US2014/0287509 (Sharei et al); US2018/0003696 (Sharei et al)]. To increase the yield of the apparatus, a number of openings for the transfer of cells may be fabricated on a chip so that there is parallel transfer though a number of pathways. The disadvantage of this approach is that it operates on the principle of one cell transferred through an opening at a time which limits the yield of the instrument. The second disadvantage is that the openings that have the size comparable with the size of the cells and even smaller than it, can be blocked by cells in the solution and other particulates.

There are some other methods for injection of DNA into cells. The biolistic technology relies of loading DNA onto the surface of nanoparticles, such as e.g. gold particles. These are transferred into plastic cartridge tubes and then loaded in a pressure "gun". The pressure "gun" ejects the gold particles on the target cells such as e.g. plant cells. Another method is direct microinjection by a pipet. This method is more commonly used for stem cells or egg cells. There is finally another method called optoinjection [D.J. Stevenson, F.J. Gunn-Moore, P. Campbell, K. Dholakia, Single cell optical transfection, J. R. Soc. Interface (2010) 7, 863-871]. In this method the wall of the cell is altered by a laser beam and it then becomes permeable to the transfer of the biological material into the cell.

It is important to determine the condition of the cell upon the completion of transfection. This is necessary to evaluate the viability of the cell, the effectiveness of the transfection and the immediate impact of the transfection procedure on the viability of the cell. It is important to optimise the transfection procedure. It is important to know the length of time when the cell membrane stays open to the introduction of the biological molecules from the ambience to the interior of the cell. It is important to know the length of time required for a cell to recover from the negative impact of the transfection procedure. It is desirable to be able to remove the subset of cells from the overall set of cells that are no longer alive following the impact of the transfection so that they do not enter into the next stage of work alongside with the viable cells. It is further more desirable to remove the subset of cells from the overall set of cells whose membrane was not opened up for the transfection so that they do not enter the next stage of work alongside with the cells that have their membrane opened up for the transfection.
One object of the present invention is to provide a device and a method that can differentiate between the cells that are not alive or no longer viable from the ones that are viable following the transfection procedure.
Another objective of this invention is to provide a device and a method that can differentiate between the cells that have not being transfected from the ones that have.
Another objective of the invention is to separate the cells following the transfection into subsets such as cells that have their membrane opened for transfection, cells that have not their membrane opened for transfection and the cells that lost their viability or died. Another objective of the invention is to provide a device and method for the transfection of cells.
Another objective of the invention is to provide a device and method for the transfection of cells by way of mechanical stimulus to the cells.
Yet another objective is to provide a device and method where the extent of the mechanical stimulus and hence the extent of the alteration to the cell membrane can be tuned to optimise the conditions of opening the cell membrane and yet achieving acceptable level of the cell viability following the transfection that are both specific to the cell type and the condition of the cell.
Another objective is to provide the device and method for transfection in the on-chip format that could be integrated with other benefits of on-chip cell processing, cell separation, cell monitoring, cell handling and the cell control.
Another objective is to increase the throughput in number of cells that could be transfected per unit of time (e.g. per second)
Another objective is to provide a device for cell transfection that is not prone to blocking.

### Summary of the Invention

The present invention addresses the need for a method and device for transfecting cells that overcomes the limitations of the prior art, by providing a microfluidic based method of physically disrupting cell membranes, that can be combined with other microfluidic processes such as cell sensing, cell separation and cell handling, and a device for carrying out the method. The method involves passing the cells in a carrier liquid along a microfluidic channel that has a cross-sectional area greater than that of the cells, and that incorporates a flow obstacle that causes the flow vector of the cells and carrier liquid in the microfluidic channel to abruptly change from a first direction to a second direction. The Applicant has discovered that such an abrupt change in flow vector of the cells in a microfluidic channel causes disruption of the cells membrane making it suitable for subsequent transfection of the cells with foreign material such as DNA (i.e. transfection-competent disruption). The flow obstacle may be a turn in the microfluidic channel, or an obstacle disposed in a straight microfluidic channel, provided that they cause an abrupt change in flow vector of the cells and the carrier fluid. As the method of the invention may be carried out in a microfluidic channel (i.e. in a chip), the handling of the cells after cell membrane disruption may be easily controlled by incorporating known in-chip cell monitoring and cell separation technologies, for example WO2017/202932 and WO2017/182599. Thus, the methods of the invention may also be employed to separate viable cells from non-viable cells, and separate viable transfection competent cells from other cells in a cell population, all of which can be carried out a single chip. In addition, the transfection of foreign material into the cell may also be carried out in the microfluidic channel by incorporating foreign material into the carrier fluid.

According to a first aspect of the present invention, there is provided a method of transfecting a population of cells, comprising the steps of:
treating the population of cells to disrupt a cell membrane of at least some of the cells; and
introducing foreign material into at least some of the cells through the disrupted cell membranes,
characterised in that the cell membranes are disrupted by passing a cell-containing carrier liquid along a microfluidic channel having a cross-sectional dimension greater than a cross-sectional dimension of the cells and comprising a flow obstacle configured to abruptly change a flow vector of the cells and/or carrier liquid in the microfluidic channel from a first direction to a second direction, whereby the abrupt change in flow vector of the cells effects transfection-competent disruption of the cell membrane of at least some of the cells due to impact of the cells with the flow obstacle and/or with other cells.

In a further aspect, there is provided a method of preparing a population of cells for transfection by physically treating the cells to disrupt the cell membrane of at least some of the cells,
characterised in that the physical treatment step comprises passing a cell-containing carrier liquid along a microfluidic channel having a cross-section dimension greater a cross-section dimension of the cells and comprising a flow obstacle configured to abruptly change a flow vector of the cells and/or carrier liquid in the microfluidic channel from a first direction to a second direction, whereby the abrupt change in flow vector of the cells effects transfection-competent disruption of the cell membrane of at least some of the cells due to impact of the cells with the flow obstacle and/or with other cells.

In a further aspect, there is provided a method of physically treating cells to disrupt the cell membrane of at least some of the cells,
characterised in that the physical treatment step comprises passing a cell-containing carrier liquid along a microfluidic channel having a cross-section dimension greater than a cross-section dimension of the cells and comprising a flow obstacle configured to abruptly change a flow vector of the cells and/or carrier liquid in the microfluidic channel from a first direction to a second direction, whereby the abrupt change in flow vector of the cells effects transfection-competent disruption of the cell membrane of at least some of the cells due to impact of the cells with the flow obstacle and/or with other cells.

In one embodiment, the method includes a step of focussing a stream of the cell containing liquid using a hydrodynamic focussing device, typically configured to provide a focussed stream comprising a core cell containing stream and a positioning stream of liquid forming a sheath around the core stream. Preferably, the core cell containing stream is disposed adjacent an outer wall of the microfluidic channel upstream of the flow obstacle. Preferably, the core cell containing stream merges with the outer wall of the microfluidic channel at or just before the flow obstacle. Methods and devices configured for in-chip hydrodynamic focussing are described in WO2017/182559.

In one embodiment, particulate matter such as beads is added into the flow of cell-containing carrier liquid to enhance disruption of the cell membranes.

In one embodiment, the cell containing fluid comprises foreign material, wherein the method includes a step of transfection of foreign material into the cells in the microfluidic channel downstream of the flow obstacle.

In one embodiment, the microfluidic channel comprises a detection zone downstream of the flow obstacle comprising a sensor configured to detect a parameter corresponding to single cells passing the sensor, and a separation zone downstream of the detection zone comprising a force generator configured to displace single cells in response to a single cell-specific parameter detected by the sensor. In this embodiment, the method includes the steps of detecting a parameter corresponding to single cells passing the sensor, and displacing single cells with the force generator in response to a single cell-specific parameter detected by the sensor.

In one embodiment, the method includes a step of detecting and separating viable cells, in which the sensor is configured to detect viable single cells.

In one embodiment, the method includes a step of detecting and separating transfection-competent cells, in which the sensor is configured to detect transfection-competent single cells.

In one embodiment, the method includes a step of detecting and separating viable transfection-competent cells, in which the sensor is configured to detect viable transfection-competent single cells.

In one embodiment, the cell-containing carrier liquid is passed along the microfluidic channel at a linear velocity of 0.1 m/s to 10 m/s, or 0.1 m/s to 5 m/s.

In a further aspect, there is provided a device for preparing a population of cells having cell membranes for transfection by physically treating the cells to disrupt the cell membrane of the cells, the device comprising:
a microfluidic channel having a cross-section dimension greater than a cross-section dimension of the cells; and
a pump operatively connected to the microfluidic channel configured to pump a cell-containing liquid through the microfluidic channel,
characterised in that the microfluidic channel comprises a flow obstacle configured to abruptly change a flow vector of the cells and carrier liquid in the microfluidic channel from a first direction to a second direction during use, wherein the abrupt change in flow vector of the cells is configured to cause transfection-competent disruption of the cell membrane of at least some of the cells due to impact of the cells with the flow obstacle and/or with other cells.

In one embodiment, the microfluidic channel comprises an abrupt turn, wherein the flow obstacle is provided by an outer wall section of the microfluidic channel at the abrupt turn.

In one embodiment, the abrupt turn in the microfluidic channel has an angle of 30°-180°, for example at least 30°, 45°, 60°, 90°, or 120°. In one embodiment the microfluidic channel comprises a plurality of abrupt turns, for example 2, 3, 4, 5, 6, 7, 8, 9, or 10. In one embodiment, at least a section of the microfluidic channel has a zig-zag shape, a sine wave-like shape, or a stepped shape. In one embodiment, the turn is provided by a Y-shaped channel, where one of the arms is an inlet section and the other arm is an outlet, wherein the stem of the Y in use is filled with cells.

In one embodiment, at least a part of the outer wall section of the microfluidic channel at the abrupt turn comprises a non-smooth cell membrane disrupting surface. The non-smooth cell membrane disrupting surface may comprise indentations, projections, or may be may comprise a corrugated or undulating surface.

In one embodiment, the device comprises a hydrodynamic focussing device configured to provide a focussed stream comprising a core cell containing stream and a positioning stream of liquid forming a sheath around the core stream, in which the core cell containing stream is disposed adjacent an outer wall of the microfluidic channel downstream of the flow obstacle.

In one embodiment, the microfluidic channel comprises a detection zone downstream of the flow obstacle comprising a sensor configured to detect a parameter corresponding to single cells passing the sensor, and a separation zone downstream of the detection zone comprising a force generator configured to displace single cells in response to a single cell-specific parameter detected by the sensor.

The sensor may be configured to detect viable cells, non-viable cells, transfection competent cells, transfection incompetent cells, and viable transfection competent cells.

In one embodiment, the microfluidic channel has a cross sectional dimension in the range of 2-2000 microns. In one embodiment, the microfluidic channel has a cross sectional dimension 1-1000 times greater than the cross-sectional dimension of the cells.

Other aspects and preferred embodiments of the invention are defined and described in the other claims set out below.

### Brief Description of the Figures

**Figure 1****:** distribution of velocities of a liquid in a straight microfluidic channel across the channel cross-section. The cell carrying liquid carries cells along the channel in this example and the flow is sustained by a pressure difference applied along the microfluidic channel.
**Figure. 2****:** embodiment of a channel with a 90-degrees change in the flow vector direction and straight walls.
**Figure. 3****:** embodiment of a channel with angle of the change in the flow vector direction of greater than 90 degrees
**Figure. 4****:** embodiment of a channel with angle of the change in the flow vector direction of less than 90 degrees
**Figure. 5****:** embodiment of a channel with a smaller width of the channel in the section 2 of the channel than in the section 1 of the channel and a 90-degree turn of the flow vector.
**Figures. 6a****,b.** Embodiment of the apparatus used to demonstrate the impactful movement of the cells towards the virtual flow obstacle
**Figure. 7****.** Embodiment with ruggedized surface of the flow obstacle.
**Figure. 8****.** Embodiment with ruggedized surface of the flow obstacle protruding into the channel and 180 degrees turn of the flow vector.
**Figure. 9****.** Embodiment of the channel with multiple abrupt turns and multiple flow obstacles.
**Figure. 10****.** Embodiment of the channel with multiple abrupt turns and multiple flow obstacles.
**Figure. 11****.** Embodiment of the channel with multiple abrupt turns and multiple flow obstacles.
**Figure. 12****.** Embodiment of the microfluidic channel with multiple flow obstacles.
**Figure. 13****.** Embodiment of the microfluidic channel with flow obstacle separated from the outer wall of the channel.
**Figure. 14****.** Embodiment of the microfluidic channel with two streams: core cell-containing stream and positioning stream creating focussed stream of cells.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "transfection" means the process by which foreign material generally nucleic acid material, is introduced into the cell. As described above, various methods are described in the literature for transfecting cells, including methods that involve physical disruption of the cell membrane to allow introduction of foreign material into the cell through the disrupted cell membrane. The methods of the invention comprise physical disruption of cell membranes to allow transfection, and generally exclude virus-mediated cell transfection (transduction). The term includes stable and transient transfection, and transfection with DNA and RNA.

As used herein, the term "foreign material" refers to the material that is introduced into the cell during the process of disruption. The foreign material is generally nucleic acid material, for example DNA or RNA, which may be naked or purified, or form part of a nucleic acid construct including a transgene and other functional components such as promotors, poly adenylation tails and Kozak sequences (cloning vectors, plasmids, expression vectors, and artificial chromosomes).

As used herein the term "Cells" means any type of cell, including mammalian cells such as white blood cells, red blood cells, bone marrow cells, immune cells, epithelial cells, nerve cells, pulmonary cells, vascular cells, hepatic cells, kidney cells, skin cells, stem cells, or bacterial and fungal cells and hybridomas. The device and methods of the invention may be employed to prepare cells for transfection, and optionally to monitor the cells to detect and/or separate transfection competent cells. The device and methods of the invention may be employed to transfect cells, and optionally to monitor the cells to detect and/or separate transfected cells. The device and methods of the invention may be employed to prepare cells for transfection or transfect cells, and optionally to monitor the cells for viable cells and optionally separate viable and non-viable cells.

As used herein, the term "Focussed stream of cell containing liquid" means a liquid containing cells in the form of a core stream containing the cells and a positioning stream that at least partially, or possibly fully, embraces the core stream. In one embodiment the cells in the core stream are focussed into a single file arrangement. In one embodiment, the cells in the core stream are aligned in the same direction. In one embodiment the core stream is positioned between the positioning stream and at least one wall of the channel. Methods and devices configured for hydrodynamic focussing cells in a liquid stream are described in WO2017/182599.

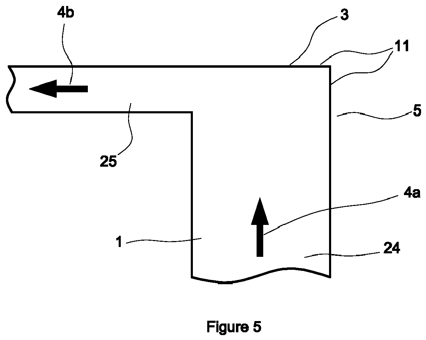

As used herein, the term "Microfluidic channel" means a channel having a cross-sectional area of less than 4 mm² and a length of at least 1mm. In one embodiment, the microfluidic channel has a cross-sectional area of less than 0.25 mm². In one embodiment, the microfluidic channel has a cross-sectional area of less than 0.01 mm². In one embodiment, the microfluidic channel has a cross-sectional area of less than 0.0025 mm². In one embodiment, the microfluidic channel has a length of at least 1.500 mm. In one embodiment, the microfluidic channel has a length of at least 2 mm. In one embodiment, the microfluidic channel has a length of at least 10 mm. Generally, the microfluidic channel is provided on a substrate such as a chip. In one embodiment, the microfluidic chip comprises a plurality of layers, for example at least 2, 3, 4, 5, 6, 7, 8, 9 or 10 layers. In one embodiment, the cross-sectional area of the microfluidic channel is constant along its length. In one embodiment, the cross-sectional area of the microfluidic channel is variable along its length. In one embodiment, the cross-sectional area of the microfluidic channel downstream of the flow obstacle is smaller than the cross-sectional area upstream of the flow obstacle (see Fig.5). In one embodiment, the cross-sectional area of the microfluidic channel upstream of the flow obstacle is smaller than the cross-sectional area downstream of the flow obstacle (see Fig.5).

As used herein, the term "flow obstacle" means a surface in the microfluidic channel that is positioned in the way of the cells and carrier fluid and which causes the flow vector of the carrier fluid and/or cells to abruptly change from a first direction upstream of the flow obstacle to a second direction downstream of the flow obstacle. The term "abrupt" should be understood to mean that the flow obstacle is configured to change the flow vector rapidly, almost instantaneously, and does not include slow, gradual changes in flow vector. Various methods and devices are described herein that incorporate flow obstacles configured to cause an abrupt change in flow vector. In one embodiment, the flow obstacle is provided a turn in the microfluidic channel, where the cells and carrier fluid impact with an outer wall of the microfluidic channel at the turn, causing the flow vector of the cells to change and in the process causing the cells to impact with the wall of the channel and with each other. The turn can typically have a change in direction of 30 to 180 degrees, and preferably 60-180, 90-180, 60-120, or 30-120 degrees. Generally, the wall of the turn where the cells impact (impact section) is flat, as opposed to curved. In one embodiment, the impact section of the wall has a non-smooth cell membrane disrupting surface, and may incorporate ridges, indentations, corrugations, or raised formations, configured to assist in cell membrane disruption when the cell impacts the wall. In one embodiment, the impact section of the wall is provided by an insert that is disposed in the microfluidic channel, in front of and adjacent the wall. In another embodiment, the flow obstacle may be provided by one or more impact barriers (for example a series of impact barriers) which may be disposed in the microfluidic channel at positions which cause the flow vector to change abruptly, and in one embodiment change abruptly several times. It will be appreciated that the term "flow obstacle" does not encompass prior art methods where cells are squeezed through channels or apertures that are undersized with respect to the cells.

As used herein, the term "transfection-competent disruption of the cell membrane" refers to disruption of the cell membrane which allows foreign material to be transfected into the cell through the disrupted cell membrane, within the microfluidic channel, or in a separate non-microfluidic process.

As used herein, the term "Detection zone" means a cross-section of the microfluidic channel at which a sensor such as an electrode pair is located. The sensor may be configured to detect AC impedance changes in the microfluidic channel caused by single cells passing the sensor, i.e. changes in impedance detected at the detection electrode. The changes may include changes in amplitude, phase, or amplitude and phase of the signal. Details of such sensors are described in the patent applications WO2017/202932 and WO2017/182599.

As used herein, the term "Separation zone" is a part of the microfluidic channel, distal of the detection zone, where cells in the fluid can be separated based on the parameter changes in the channel caused by the cells in the detection zone. The separation zone generally includes a force generator operably connected to the senor and configured to exert a force on the cells in response to signals from the detection zone, to separate the one or more particulates from the stream of fluid. Examples of suitable force generators for use in cell sorting apparatus are well known in the art and described for example in Wyatt Shields et al ("Microfluidic Cell Sorting: A Review of the Advances in the Separation of Cells from Debulking to Rare Cell Isolation", C. Wyatt Shields IV et al, Lab Chip. 2015 February 16, 15(5): 1230-1249). In one embodiment, the device will typically include a processor operably connected to the at least one sensor and the force generator and configured to actuate the force generator in response to a signal received from the sensor. The actuating signal may be pre-programmed into the processor, and may vary from cell type to cell type.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

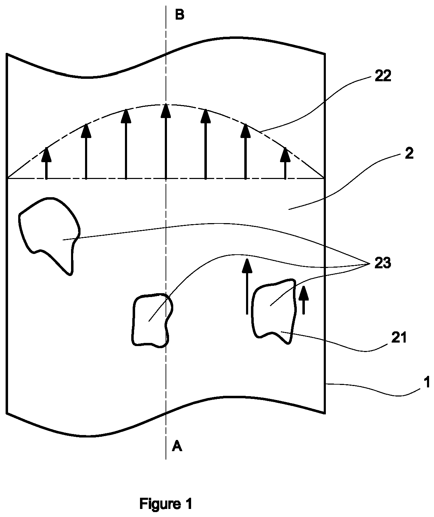

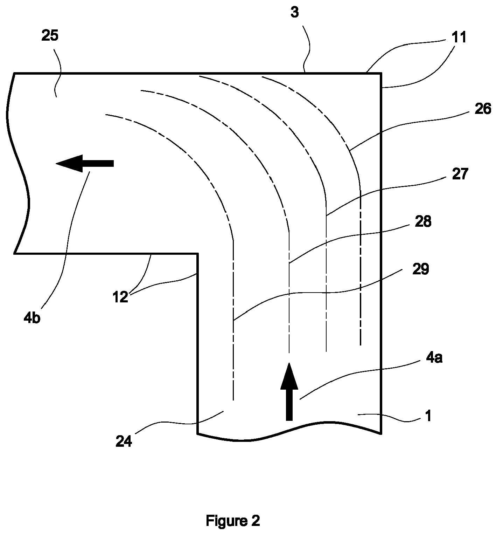

To best understand the invention, one needs to consider a flow of cells suspended in a cell-containing liquid carrying the cells in a microfluidic channel. In our case the microfluidic channel has cross-section that is broadly comparable with the size of the cells, and yet larger than the size of the cell, e.g. 1 to1000 times larger than the size of the cell. These figures are given here as rough indication, and the dimensions of the channel outside this range can be also deployed; for example, the channel could 2000 times larger than the size of the cells. The microfluidic channel may also be referred to as the channel for shortness. The mass density of the cells (i.e. mass of the cell divided by the volume of the cell) is not exactly equal to the mass density of the carrying liquid, even though these two densities are normally broadly comparable. The liquid carrying cells could be any physiological saline buffer e.g. PBS (phosphate saline buffer), TRIS buffered saline, DMEM (Dulbecco's Modified Eagle's Medium). Various other liquids commonly used in cell laboratory, pharmaceutical and medical work could also be deployed as the cell carrying liquid. The difference in densities of the cells and of the liquid is defined by the type of the cells and the type of the cell carrying liquid. Due to the density difference, the cells may tend to move closer either to the top or to the bottom part of the channel. If the flow of the liquid in a channel is driven by a pressure differential along the channel, the linear velocity of the liquid will vary across the channel. The velocity is normally highest at the central area of the channel cross-section and lowest along the walls of the channel, although the details are determined by the geometry of the cross-section of the channel. If the flow is sustained e.g. by electroosmotic forces as opposed to a mechanical pressure differential applied along the channel, the distribution of the flow across the channel may be different but nonetheless, usually the linear velocity of the flow varies across different points in the channel's cross-section. This may result in the difference in the flow velocities along different sides of a cell as shown schematically by two vectors on both sides of a cell marked by numeral (21) in the Fig 1 and consequently may result in a hydrodynamic force acting on the cell having a component perpendicular to axis of the channel A-B. Fig. 1 shows the flow velocity at different points of the channel by the flow velocity profile (22) schematics of the cell carrying liquid (2). The length of the arrows of the flow velocity profile is indicative of the linear velocity of the cell carrying liquid (2) at the respective part of the channel marked by the arrow. Fig 1 also shows three cells marked by numeral (23) travelling in the channel as an example. The flow velocity is shown in the vicinity of one of the cells labelled by numeral (21). The flow velocity is shown by two vectors to indicate that the velocity at the right edge of that particular cell (21) is lower than the one at the left edge. The value of the hydrodynamic force acting on the cell in the flow of the cell-carrying liquid is determined by the flow of the liquid in the channel and its position within the channel. The combination of the gravity force, buoyancy of the fluid and the hydrodynamic force may result in preferential positioning of the cell at some parts of the cross-section of the channel, resulting in aggregation of the cells e.g. mainly towards the centre or mainly towards the lower (floor) side of the channel or towards the upper (ceiling) side of the channel. The preferential positions of the cells are determined by the shape of the channel, hydrodynamic characteristics of the cells (density, shape) and of the carrying liquid and also by the orientation of the channel with respect to the gravity direction. We have found that if the flow in the channel changes, and if such a change is abrupt, the cells will not immediately re-adjust to the new conditions of the flow. Furthermore, we have found that if the flow direction changes suddenly/abruptly at a given point along the channel, the cells may overshoot and collide with the wall of the channel. If the density of the cells is high, they may also collide with other cells, e.g. cells that have bounced from the wall of the channel may collide with the ones moving towards the wall. This is shown schematically in Fig. 2 that depicts location of an abrupt change from the first flow direction represented by flow vector 1 and marked by numeral (4a) in the channel section 1 (numeral 24) to the flow direction 2 represented by the flow vector 2 and marked by numeral (4b) in the channel section 2 (numeral 25). We show schematically four pathways for cells positioned along different parts of the cross-section of the channel section 1 (24). These four pathways are shown by dashed lines and marked by numerals (26), (27), (28), (29). The cells are not shown in Fig. 2 for clarity of the diagram but it is understood the channel is filled with the liquid carrying the cells and the cells themselves. We demonstrate with Fig. 2 that the cells travelling closer to the right edge of the channel section 1 (24) collide with the outer wall of the microfluidic channel proximal the corner. The outer wall in Fig. 2 is marked by the numeral (11). The cells collide with the section of the outer wall that is forming the beginning of channel section 2. This is shown by the dashed lines of the pathways 1 (numeral 26) and 2 (numeral 27) that make contact with the outer wall of the channel section 2 (25). The impact section of the channel wall located on the outer wall of the channel that is marked in Fig. 2 by numeral (3) thus represents the flow obstacle to the cells. The outer wall (11) is the wall along the fluidic pathways of greater radius of curvature. The opposite wall, i.e. the wall along the fluidic pathways of smaller radius of curvature, is also marked in Fig. 2 for clarity and is marked by the numeral (12). The impact section of the channel outer wall forming the flow obstacle (3) in Fig. 2 is the section adjacent the corner along the outer wall that is facing the flow vector 1 (4a), i.e. the flow vector of the upstream channel section 1 (24).

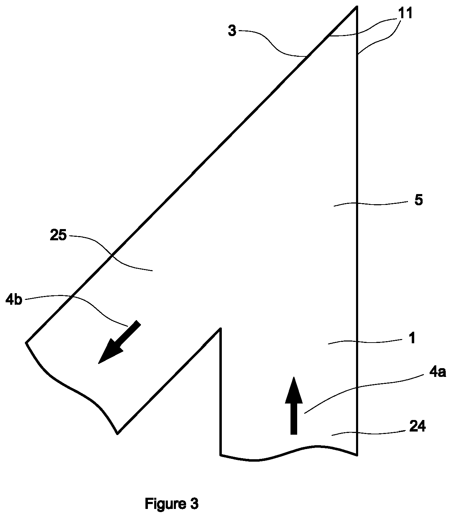

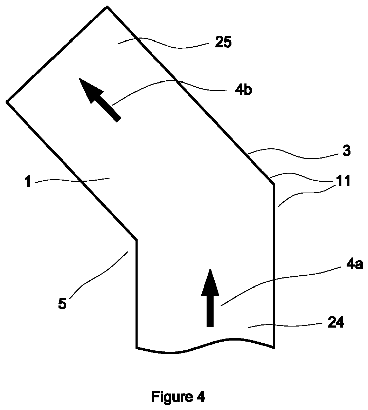

In this document we shall call this abrupt change in the flow direction along the length of the channel, the change in the flow vector. The term change in the flow vector rather than change in the flow direction will be used to avoid misleading analogy of a movement in one direction that first stops and then reverses. In contrast, here we consider flow where the line representing the direction of the channel abruptly changes from one orientation in space to another one. With reference to Fig. 2 we can say that the flow changed from the flow vector 1 (4a) in the channel section 1 (24) to the flow vector 2 (4b) in the channel section 2 (25). Several examples of such locations enabling the changes in the flow vectors are shown in Figs 3, 4. The direction of flow vector changes by 90 degrees is Fig 2, by some 140 degrees in Fig 3 and by 50 degrees in Fig. 4. The channel sections 1 and 2 in Figs 2-4 are straight, each having opposite parallel walls. These are shown straight as convenient embodiments. The sections do not have to be straight; and embodiment with non-parallel opposite walls can also be constructed.

The cross sections of the channel on both sides of the location of the change of the flow vector do not have to be identical as shown by embodiment in Fig. 5. This figure shows examples of the channels having different widths on both sides of the point of flow vector change. In this case the cross-section of the channel downstream from the point of the flow vector change (25) is smaller compared the one upstream (25). This may have advantage that as the cross-section of the channel is reduced, the linear velocity of the flow increases. This means that the cells are subjected to greater pressure and greater shear stress impact at the point of the flow vector change at the junction of the sections 1 and 2, i.e. at the flow obstacle (3).

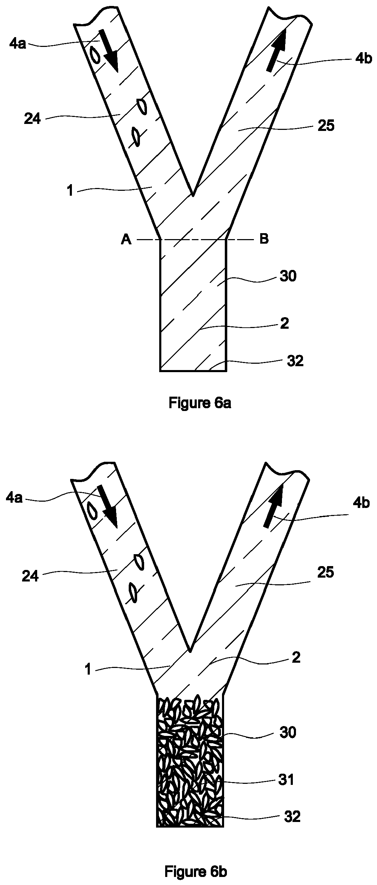

It is difficult to observe directly collision of the cells with the wall of the channel as the process of collision is fast. To confirm that the cells do reach the wall and collide with it at the point of the change of the flow vector, we have performed the following experiment. We have passed the cells of yeast cells (*Saccharomyces cerevisiae*) via a Y -channel with the central section 3 (30) blocked off by a termination (32) as shown in Fig. 6a. The cells travel from the channel section 1 (24) with the flow vector 1 (4a) into the channel section 2 (25) with the flow vector 2 (4b). In this experiment there is no systematic flow in the channel section 3 (30) as it is intentionally blanked off by us by a termination (32). Therefore, the channel section 3 (30) forms an appendix or a cul-de-sac. If the cells cross the imaginary line separating the channel sections 1 (24) and 2 (25) from the channel section 3 (30) that is marked by letters AB in Fig. 6a, this would imply that they were to collide with the wall that would otherwise be there in the case of the structures of e.g. Fig. 3, 4 or 5, that is the flow obstacle section of the channel wall. To achieve the conditions of the experiment as described above, we have first primed all the channel sections with a carrying liquid without any cells. This moment shown in Fig. 6a with the channel sections 1 (24), 2 (25), 3 (30), all filled completely by a carrying liquid. We then pumped the cells at the concentration of 1 million cells/ml and the flow of 200 ul/min into the channel section 1 (24). This is again shown schematically in Fig. 6a where the first few cells moving into the channel section 1 (24) are depicted, and yet there are not cells in the channel section 2 (25). The channels have width of 200 micrometres and depth of 30 micrometres. Once the cells are added into the flow, we have observed accumulation of the cells in the central section of the channel. This accumulation is shown in Fig. 6b. After a length of time the channel section 3 (30) becomes filled with aggregation of the cells (31) and the mean density of the cells in the channel section 2 (25) becomes equal to the one in the channel section 1 (24).
If cells move with velocity of 5-10 m/s making an impact against a solid surface or against another cell, or if they move in a shear mode movement with the same velocity against solid obstacles, they could be lysed, i.e. this could result in irreversible rupture of cell membrane [D. Boal, Mechanics of the Cell, Cambridge University Press, Cambridge, 2003; L.J. A. Beckers, M. Baragona, S. Shulepov, T. Vliegenthart, A.R. van Doom, Mechanical Cell Lysing Device, Proc. 14th International Conference on Miniaturized Systems for Chemistry and Life Sciences, 3-7 October, 2010, Groningen, The Netherlands, p.85-87]. The exact velocity required for rupturing the cell membrane depends on type of cells. The figure of the 5-10 m/s is representative of more difficult-to-lyse organisms like e.g. bacteria. Generally, velocities below that range are required to achieve reversible damage to the cell membrane as necessary for the successful transfection.

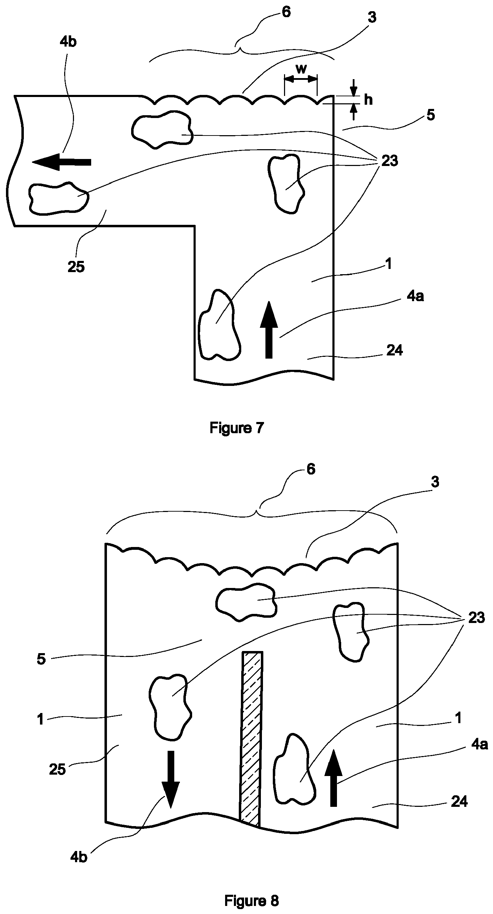

To facilitate the reversible damage to the cell membrane and the transfection, in some embodiments the impact section of the wall of the microfluidic channel forming the obstacle (3), is ruggedized. One such embodiment is shown in Fig. 7 where the impact section of the wall comprises a number of vertical protrusions. The separation between the protrusions (w) and the height (h) of the protrusion are preferably smaller than the characteristic size of the cell. The protrusions form the cell-membrane disrupting surface (6).

Fig. 8 shows another embodiment of the chip with the rotation of the flow vector by 180 degrees from the channel section 1 (24) to the channel section 2 (25). In this embodiment the wall of the impact section of the channel is not flat but is rather protruding into the channel. Furthermore, the part of the wall forming the flow obstacle is ruggedized to enhance the impact of the cells collision with the wall thus creating the cell membrane disrupting surface (6).

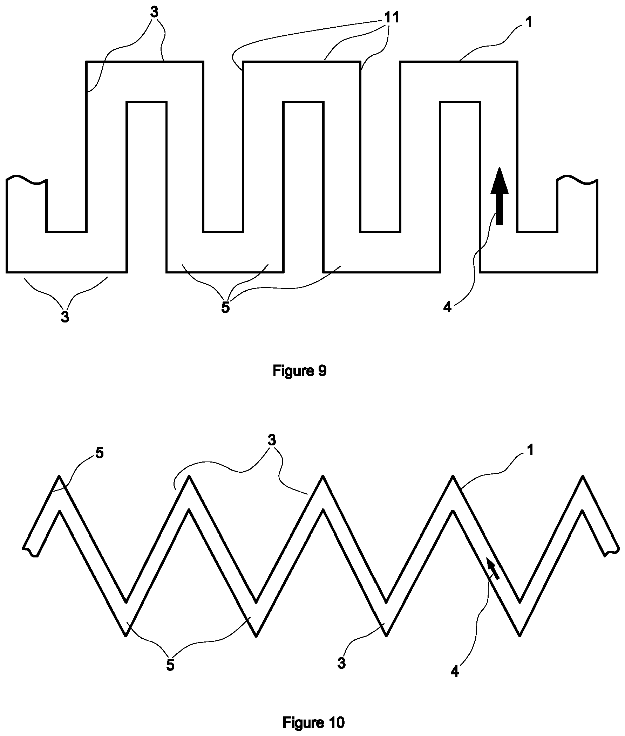

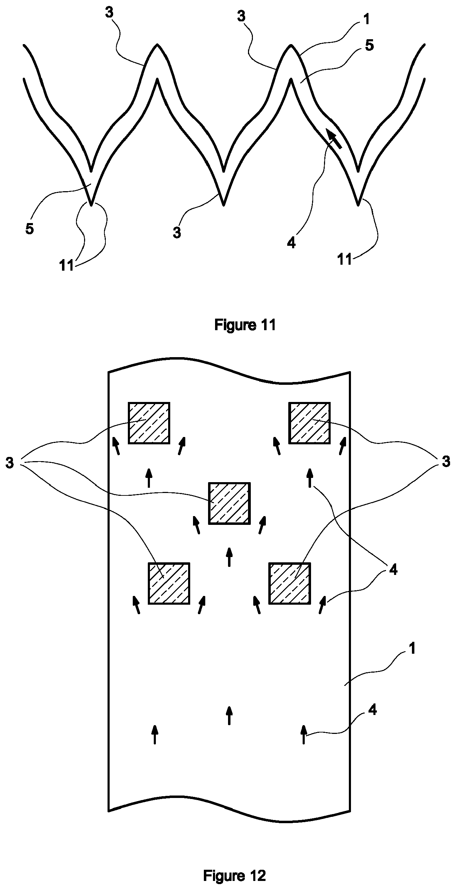

Fig. 9, 10, 11 represent embodiments where the flow vector along the microfluidic channel (1) changes not just a single time as in embodiment shown in the previous figures but rather changes a number of times. Flow vector (4) in one of the channel sections is shown by an arrow in Figs 9, 10, 11. All channel sections are not explicitly indicated by numeral for clarity of the drawing. Only some flow obstacles, channel sections and abrupt turns are marked by the numerals for the clarity of the drawing. The numerals used are the same as in previous figures. The sections of the channels do not have to be straight as shown in Fig. 11. The abrupt turns between consecutive channel sections do not have to be all identical.

The area of the channel at the point of the change of flow vector can comprise a number of flow obstacles (3) as shown in Fig. 12. These flow obstacles enhance the impact of the cells as they move to re-adjust to the new flow vector along the channel. In this embodiment the flow obstacles can be considered as positions of multiple local abrupt changes in the flow vector rather than change in the overall flow vector in the microfluidic channel. At proximity to these obstacles, the cells deviate from the flow of the cell-containing carrying liquid so that their instant velocity becomes significantly different from that of the carrying liquid and as a result they impact into the flow obstacles (3) in normal-like movement against the wall of flow obstacle (i.e. near normal-like impact against the surface) or in tangential-like movement against the surface of the flow obstacle (i.e. near shear-like movement along the surface) or in some combination of these two modes of movement. The local flow vectors (4) are shown in the channel by arrows.

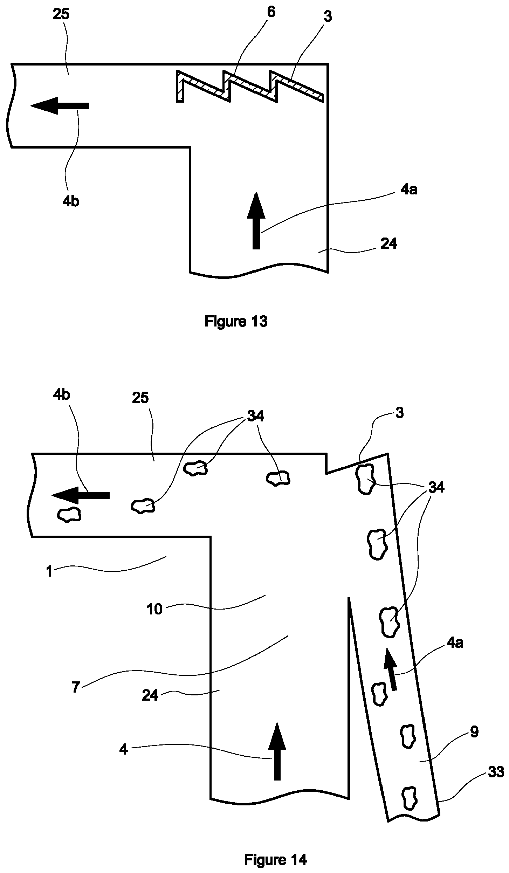

The obstacles could be positioned along the channel wall and yet be separate from the channel wall. In this case the impact section of the channel wall that also serves as integrated flow obstacle is replaced for a standalone flow obstacle. This embodiment is shown in Fig. 13. The shape of the flow obstacle is chosen to increase the change in the flow vector direction and increase the flow vector inhomogeneity to increase the events of cells impacting with each other and with the flow obstacle. The same numerals are used as in earlier figures.

The microchannel structure does not need to be planar. All the previous embodiments show the structures where the flow vectors on both sides of the point of the flow vector change are positioned in the same plane. They do not have to be positioned in the same plane. Three-dimensional structures are also possible. These will not be shown for brevity. The cells can be forcefully confined to the area of the impact section of the channel using e.g. sheath liquid and injection of the cells suspended in sample fluid into the flow of the sheath liquid that favours movement of the cells towards the impact section of the channel. Fig. 14 shows such an embodiment. In this embodiment the cells are injected into the microfluidic channel section 1 (marked by numeral 24) via a separate injection channel (33). The injection channel (33) merges with the channel section 1 (24) proximal the flow obstacle. In this embodiment the flow obstacle (3) forms part of the wall of the channel. This does not have to be always the case and one could construct embodiments with the flow obstacle being separate from the wall as in Figs. 12, 13. In this embodiment we therefore create focussed stream (8) of cells (34) guided and enveloped on the left by the positioning stream (10) injected via the channel section 1 (24). Clearly, other configurations merging two streams: the cell containing stream (9) and the positioning stream (10), are possible to create a focussed stream of cell containing liquid transporting the cells towards the flow obstacle. We used the word sheath fluid to describe positioning stream. The word sheath fluid (sheath liquid) is used in the Applicants Patent Application WO2018/182599..

In our embodiments the width of the channels is in the range of 1 to 2000 microns, the height of the channels is in the range of 1 to 2000 microns. These figures are given as indications of the cross-sectional dimensions of the channel. One should keep in mind that although the rectangular cross-section of the channels is common, they do not have to be of rectangular or circular cross-section, and instead could have the cross section of e.g. a polygon-like or an ellipse-like shape. The length of the channel is typically in the range of 0.1 mm-500 mm although the dimensions outside this range are also possible. The pressure applied to the channels is in the range of 2 Bar. The typical linear flow velocity in the channels is in the range of 0.1 m/s-10 m/sec.

In our device we could use a detection zone positioned downstream from the flow obstacle. The detection zone could be equipped with sensors to establish the condition of a cell. The cells with intact membrane will have different electric characteristics from the ones where the membrane was altered by the flow obstacle. We describe in details the methods and apparatus for measurement of the status of the cell and in particular measurements of status of the cell membrane in the patent applications WO2017/182599 and WO2017/202932. For example, one could readily determine the size of the cells and establish if they are dead or alive on the basis of their electrical characteristics by variable frequency AC measurements. The device could also be equipped with the means for separating the cells following the procedure for the alteration of the cell membrane or following the procedure of the transfer of the biological material across the altered membrane (transfection). For the separation, the apparatus could be equipped with a separation zone downstream of the detection zone comprising a force generator configured to displace single cells in response to a single cell-specific parameter detected by the sensor. The examples of the force generators, the detection zone and the separation zones are given in patent applications WO2017/182599 and WO2017/202932, that are included in this application in their entirety.

As appreciated by those skilled in the art, the apparatus is also equipped with pressure sources/pumps to establish the flow of cells along the microfluidic channel and various devices for monitoring the flow rate, the pressure, electronics for detecting individual cell, separating them into different streams, etc. These are not shown in the figures for brevity. Some of these features are described in detail in patent applications WO2017/182599 and WO2017/202932, that are included here in their entirety.

It should be appreciated that the flow obstacles that are normally made of the same material as the walls of the microfluidic channel for convenience of fabrication, could also be made of a different material if so required.

It should be appreciated that particulate matter such as solid or semi-solid beads could be added into the cell containing carrier liquid to enhance the disruptive impact of the flow obstacles on the cell membrane,

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method of transfecting a population of cells, comprising the steps of:
treating the population of cells to disrupt a cell membrane of at least some of the cells; and
introducing foreign material into at least some of the cells through the disrupted cell membranes,
**characterised in that** the cell membranes are disrupted by passing a cell-containing carrier liquid along a microfluidic channel having a cross-sectional dimension greater than a cross-sectional dimension of the cells and comprising a flow obstacle configured to abruptly change a flow vector of the cells and/or carrier liquid in the microfluidic channel from a first direction to a second direction, wherein the abrupt change in flow vector of the cells is configured to cause transfection-competent disruption of the cell membrane of at least some of the cells due to impact of the cells with the flow obstacle and/or with other cells.

2. A method according to Claim 1, in which the microfluidic channel comprises an abrupt turn, wherein the flow obstacle is provided by an outer wall section of the microfluidic channel at the abrupt turn.

3. A method according to Claim 2, in which at least a part of the outer wall section of the microfluidic channel at the abrupt turn comprises a non-smooth cell membrane disrupting surface.

4. A method according to Claim 2, in which the abrupt turn in the microfluidic channel has an angle of at least 45°.

5. A method according to any of Claims 2 to 4, including a plurality of abrupt turns.

6. A method according to any preceding Claim, including a step of focussing a stream of the cell containing liquid using a hydrodynamic focussing device configured to provide a focussed stream comprising a core cell containing stream and a positioning stream of liquid forming a sheath around the core stream, in which the core cell containing stream is disposed adjacent an outer wall of the microfluidic channel upstream of the flow obstacle.

7. A method according to any preceding Claim, in which the cell containing fluid comprises foreign material, wherein the process includes a step of transfection of foreign material into the cells in the microfluidic channel downstream of the flow obstacle.

8. A method according to any preceding Claim, in which the microfluidic channel comprises a detection zone downstream of the flow obstacle comprising a sensor configured to detect a parameter corresponding to single cells passing the sensor, and a separation zone downstream of the detection zone comprising a force generator configured to displace single cells in response to a single cell-specific parameter detected by the sensor, in which the sensor is configured to detect viable single cells or single cells having disrupted cell membranes.

9. A device for preparing a population of cells having cell membranes for transfection by physically treating the cells to disrupt the cell membrane of the cells, the device comprising:
a microfluidic channel having a cross-section dimension greater than a cross-section dimension of the cells; and
a pump operatively connected to the microfluidic channel configured to pump a cell-containing liquid through the microfluidic channel,
**characterised in that** the microfluidic channel comprises a flow obstacle configured to abruptly change a flow vector of the cells and/or carrier liquid in the microfluidic channel from a first direction to a second direction during use, wherein the abrupt change in flow vector of the cells is configured to cause transfection-competent disruption of the cell membrane of at least some of the cells due to impact of the cells with the flow obstacle and/or with other cells.

10. A device according to Claim 9, in which the microfluidic channel comprises an abrupt turn, wherein the flow obstacle is provided by an outer wall section of the microfluidic channel at the abrupt turn.

11. A device according to Claim 10, in which at least a part of the outer wall section of the microfluidic channel at the abrupt turn comprises a non-smooth cell membrane disrupting surface.

12. A device according to Claim 10 or 11, in which the abrupt turn in the microfluidic channel has an angle of at least 45°.

13. A device according to any of Claims 10 to 12, including a plurality of abrupt turns.

14. A device according to any preceding Claim, including a hydrodynamic focussing device configured to provide a focussed stream comprising a core cell containing stream and a positioning stream of liquid forming a sheath around the core stream upstream of the flow obstacle.

15. A device according to any preceding Claim, in which the microfluidic channel comprises a detection zone downstream of the flow obstacle comprising a sensor configured to detect a parameter corresponding to single cells passing the sensor, and a separation zone downstream of the detection zone comprising a force generator configured to displace single cells in response to a single cell-specific parameter detected by the sensor, in which the sensor is configured to detect viable single cells or single cells having disrupted cell membranes..
